# EUROPEAN PATENT APPLICATION

(11) **EP 0 531 882 A2**
(43) Date of publication of application: **17.03.1993**
(21) Application number: 92115055.3
(22) Date of filing: 03.09.1992
(51) Int. Cl.: G06F 15/42

(54) **A method for providing a summary display of data in an information based computer system**

(30) Priority: 09.09.1991 US 756690
(71) Applicant: EMTEK HEALTH CARE SYSTEMS INC., Tempe, Arizona 85282 (US)
(72) Inventor: Brimm, John E., Scottsdale, Arizona 85258 (US); Paul, Ronald E., Phoenix, Arizona 85020 (US)
(74) Representative: Dunlop, Hugh Christopher

(57) **Abstract**

A class of data to be displayed (402, FIG. 24) is first retrieved from a memory means (12, FIG. 1) of the information based computer system. A summary rule (403, FIG. 24) for the particular class of data is then retrieved from the memory means. The steps of retrieving a class of data and the summary rule for that class are then repeated for a second class of data (405). A summary level is selected (406) to be used by the summary rules to summarize the data. The data is then summarized (408) and displayed (409).

## Description

### Field of the Invention

The present invention relates, in general, to a information based computing system and, more particularly, to a method for providing a summary display of data in an information based computer system.

### Background of the Invention

In a preferred embodiment, the present invention relates to an automated clinical records management system. Such an automatic system has utility, for example, in a hospital based patient record keeping system. Patient record keeping systems are used for maintaining a wide variety of separate, often interrelated, types of medical records concerning patients.

Hand written patient record keeping systems have evolved through many years of careful refinement and enhancement into systems which maintain a detailed manual record of medical information concerning each patient. To meet the needs of different hospital entities (such as doctors, nurses, pharmacy, accounting, laboratory, etc.) a manual record keeping system often requires that one piece of information be entered into multiple records.

In a typical manual patient record keeping system a patient chart, usually in the form of a notebook, is maintained at the nursing station for each patient. The notebook is divided into a plurality of individual tabbed sections, such as Physicians Orders, Kardex, Nursing Care Plan, Nursing Assessment, and Laboratory.

Each of the above sections is further subdivided into a number of forms. The forms are those which are appropriate to the individual patient and/or such patient's physician. For example, within the Laboratory section there may appear forms for chemistry, hematology, blood gas, and microbiology.

In addition, a "flowsheet" chart is usually kept at the patient's bedside. On the "flowsheet" chart there are individual areas for medications records, vital signs, intake/output, laboratory results, and other categories which are dependent upon the patient's affliction, such as intravenous (IV) drips.

The flowsheets are often a type of spreadsheet arranged by a progression of time versus a particular parameter. Each of the time/parameter intersections form a cell.

As an alternative to the manual record keeping procedures, automated procedures, such as those described in the inventions listed in the Related Inventions section, provide the health care provider with information in a more convenient, faster, and economical fashion.

However, there is still a need to provide the busy health care provider with a faster, more efficient manner of locating information which is of particular interest. Presently, the health care provider would have to search all of the records of a patient to see if there is any new data. In addition, this search would have to be conducted for each patient.

While automated systems have facilitated the management of patient records by: eliminating unnecessary paper work; reducing errors associated with paper work; and providing rapid access to complete medical records, they do not provide an efficient means for the assessment of patient conditions.

When a patient's data is retrieved from the computer, there is a large volume of information which may or may not be relevant to the health care provider's diagnosis. For example, if a portion of the diagnosis is based on flow sheet records for the past three days, the health care provider must review hourly records for each parameter of interest for three days. These results must then be interpreted with respect to what is normal for that patient. When the review requires several week of data, the task becomes much more complicated. This is compounded further when other records must be consulted with the flowsheet to reach a diagnosis.

To improve the productivity of the health care provider and improve patient care, it would be desirable to provide an information system that would reduce the time needed to review a patient's records.

It would also be desirable to have an information system which organizes data into summaries.

Furthermore, it would be desirable to have an information system which organizes data into levels of summaries wherein a top level summary would contain the most critical information and lower level summaries would contain progressively more information.

Additionally, it would be desirable to have an information system which organizes data into levels of summaries which may be viewed in varying time intervals.

It would also be desirable to be able to move directly from one time interval to another.

### Summary of the Invention

A method of displaying data is described wherein a class of data to be displayed is first retrieved from a memory means of the information based computer system. A summary rule for the particular class of data is then retrieved from the memory means. The steps of retrieving a class of data and the summary rule for that class are then repeated for a second class of data. A summary level is selected to be used by the summary rules to summarize the data. The data is then summarized and displayed.

### Brief Description of the Drawings

FIG. 1 is a block diagram illustrating a data processing system incorporating the present invention;
FIG. 2 is a graphical representation of a screen display representing a VITALS form in a FLOWSHEET section of a patient record;
FIG. 3 is a first level hierarchical summary form of FIG. 2 illustrating one embodiment of the present invention;
FIG. 4 is a second level hierarchical summary of the form of FIG. 3;
FIG. 5 is a graphical representation of a screen display representing an I&O form of the FLOWSHEET section of the patient record;
FIG. 6 is a first level hierarchical summary form of FIG. 5 illustrating one embodiment of the present invention;
FIG. 7 is a combination representation of the VITALS and I&O forms of the FLOWSHEET section;
FIG. 8 is a summary of the data of FIG. 7;
FIG. 9 is a graphical representation of a screen display representing a LABS form of the FLOWSHEET section of the patient record;
FIG. 10 is a summary form of the data of FIG. 9;
FIG. 11 is a graphical representation of a screen display illustrating an ASSESS form of an ASSESSMENT section;
FIG. 12 is a summary form of the data of FIG. 11;
FIG. 13 is a graphical representation of a screen display illustrating a MEDS form of the FLOWSHEET section;
FIG. 14 is a summary form of the data of FIG. 13;
FIG. 15 is a second embodiment of a summary form of the data of FIG. 2;
FIG. 16 is a first level summary form of the data of FIG. 5;
FIG. 17 is a second level summary form of the data of FIG. 5;
FIG. 18 is a third level summary form of the data of FIG. 5;
FIG. 19 is a fourth level summary form of the data of FIG. 5;
FIG. 20 is a second level summary form of the data of FIG. 11;
FIG. 21 is a shift time level summary of the third level summary form of FIG. 18;
FIG. 22 is a summary display of the data in FIG. 16 wherein specific parameters have been selected;
FIG. 23 is a block diagram of a flow chart of a process used to generate the user interface; and
FIG. 24 is a block diagram of a flow chart of a process used to summarize the data.

### Detailed Description of the Drawings

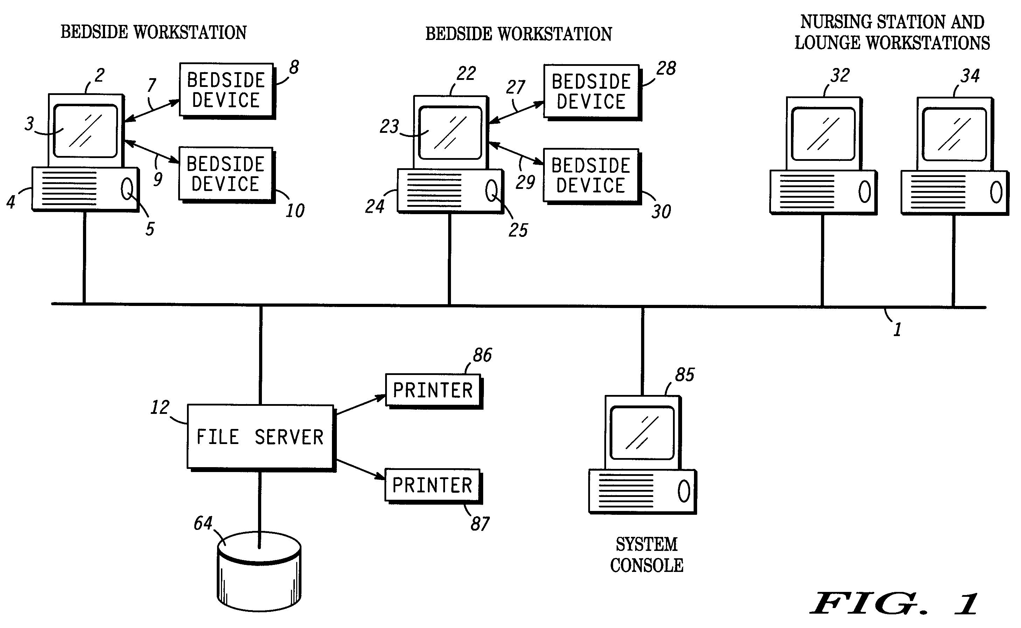

Referring initially to the block diagram of FIG. 1, a data processing system is illustrated. FIG. 1 shows a distributed computer system comprising a plurality of workstations 2, 22, 32, 34, and 85 coupled to a local area network (LAN) 1.

The system is typically installed for use in a nursing care unit, such as an intensive care unit, in a hospital or clinic. Terminals 2 and 22 are located at the patient bedside. One terminal may be dedicated to the use of a single patient, or it may be used for multiple patients. Terminals 32 and 34 may be located at a nursing station or nurse/physician lounge area. Terminal 85 is the system console which is used by a system administrator to configure and maintain the system and to provide additional services, such as displaying system status and error messages, archiving, and performing diagnostics.

Each bedside workstation or terminal, such as terminal 2, includes a video display unit with a viewable screen 3 for displaying information to the viewer; a housing 4 containing computing, data storage, and communications equipment and having associated with it a keyboard and pointing device such as a mouse 5; and connections 7 and 9 to one or more bedside devices 8 and 10. Bedside devices 8, 10, 28, and 30 may take the form of patient monitoring equipment suitable for the patient undergoing care, such as an EKG monitor, respiratory monitor, etc. Bedside terminal 22 may be coupled to a different set of bedside devices than those coupled to terminal 2.

The nursing station or lounge terminals 32 and 34, and system 85, may be identical to those used in the patient care unit but without the bedside device connections, or they may comprise slightly different equipment (e.g. personal computers) so long as they provide similar functionality.

Also coupled to LAN 1 is a file server 62 and associated storage device, here a disc storage device 64. File server 62 provides controlled access by workstations 2, 22, 32, 34, and 85 to write information to and to read information from disc storage device 64.

Optionally, interfaces (not shown) may be used to couple various peripherals to LAN 1. For example, remote access modems may be coupled to one of such interfaces to provide access to the system from remote terminals (not shown) located elsewhere in the hospital or located off-site, such as at a physician's office or residence. Remote access may also be employed to diagnose system problems from an off-site facility. A laboratory system may be connected to an interface to permit the communication of laboratory information between the laboratory system and the clinical management system. An order communication system may be coupled to an interface to permit orders to be communicated from the system to other hospital systems (e.g. pharmacy or laboratory) and vice versa. An archival storage device may be coupled to an interface to permit any information stored in the system to be archived on suitable media, such as magnetic tapes or optical discs.

Printers 86 and 87 are coupled to file server 12 to allow patient information to be printed for the convenience of hospital personnel and to maintain a suitable legal record of all observations, orders, parameter readings, care plans, and other pertinent information regarding the monitored patients. Printers 86 and 87 may be any suitable printers such as, for example, laser printers or high speed dot matrix printers. A printer may optionally be coupled to the bedside terminal and/or the terminal at the nursing station or lounge, if desired.

In operation, the system user, typically a nurse or physician, conducts a dialog with the system through the use of a keyboard, mouse, light-pen, touch-pad, trackball, or other appropriate means for entering information. "Icons", screen-sensitive areas, or the equivalent, or any combination thereof which is appropriate to the end application, may also be provided.

The user provides information to or queries the system by means of the keyboard and/or pointing device, and receives information from the system by means of information displayed on the screen and/or through audible signals which could include speech synthesis.

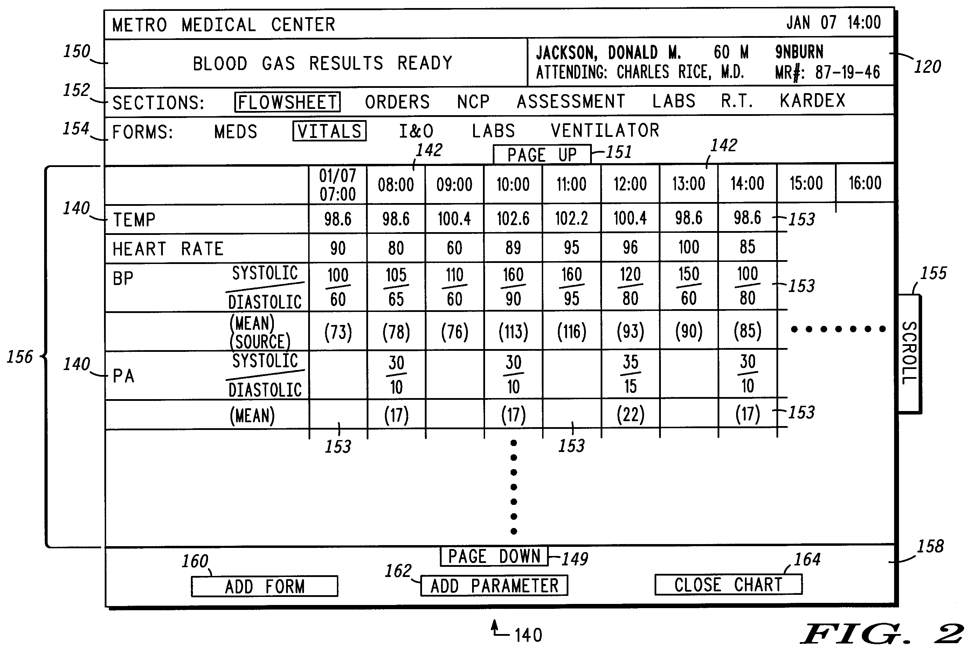

Referring to FIG. 2, a graphical representation of a screen display, generally designated 140, representing a VITALS form in a FLOWSHEET section of a patient record is illustrated. Screen 140 consists essentially of a demographic data area 120, a message area 150, a sections area 152, and a forms area 154. Demographic area 120 contains the patient's name, attending physician, age, sex, room, etc. Message area 150 provides messages to the health care provider, such as "Blood Gas Results Ready". Sections area 152 is used to select the section of a patient file which is to be viewed. In FIG. 2, the Flowsheet section has been selected as demonstrated by the reverse video highlighting. Each Section has different types of forms. The forms listed in area 154 of FIG. 2 are those associated with the Flowsheet section. The Vitals form has been selected as demonstrated by the reverse video highlighting.

Area 156 is the area where the form, or a portion thereof, selected in area 154 is displayed. In FIG. 2, various vital sign parameters, such as temperature (TEMP), Heart Rate, Blood Pressure (BP), and Pulmonary Arterial Pressure (PA), are illustrated as row headers 140. Area 156 has two dimensions: parameters (shown in the first column) and time (shown as column headers 142). At the intersection of each parameter/time pair, is a cell 153 in which data may be displayed. In FIG. 2, the time dimension is divided into hours. It should be noted that the time dimension may be varied to show more or less frequent intervals. In addition, each parameter does not necessarily have an entry for each time interval. For example, the PA data is recorded every other hour.

Area 158 provides an area for various functions to be performed. For example, FIG. 2 provides "buttons" for adding a form (160), adding a parameter (162), or closing a chart (164). FIG. 2 also illustrates data movement buttons such as a page up button 151, a page down button 149, and a scroll button (155).

Before describing the summarization in detail, some background material is necessary. Each piece of data has associated therewith a classification. When a summary is to be performed, the format of that summary display is determined by a summary rule for the class of the data being summarized. Further, each summary rule contains one or more summary levels used to summarize the data. Data may also be summarized in two dimensions, the time dimension or a categorical dimension. Therefore, the summarization of data depends upon the classification of the data, the dimension of the summary, and level of summary.

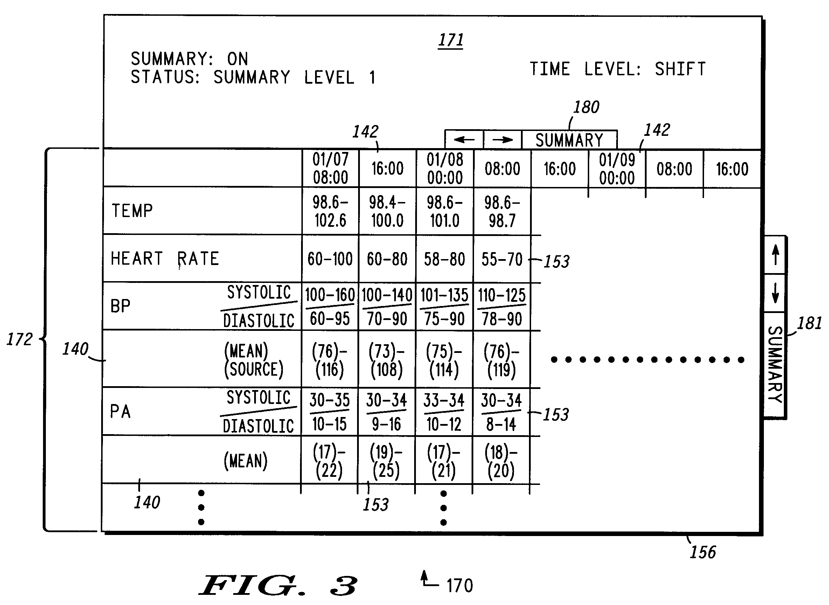

Referring now to FIG. 3, a first level hierarchical summary form, generally designated 170, embodying the present invention is illustrated. Screen 170 may be obtained in any of several standard methods in the art such as defining a function key on a keyboard to call the summary, providing a summary button on the screen, or having a keystroke command.

The summary of FIG. 3 is a time summary (i.e. the time parameter has been collapsed from one hour increments to eight hour increments). Form 170 is displayed in area 156 of the display described in FIG. 2. Form 170 is divided into two basic portions: a descriptive portion 171 and a data portion 172. In descriptive portion 171, the Summary is shown as being "ON"; the Status is "SUMMARY LEVEL 1"; and the Time Level is "SHIFT". Also provided in this example of screen 170 is a summary button 180 (to summarize in the time dimension) and a summary button 181 (to summarize in the parameter dimension).

Data portion 172 provides the summary data. The data is summarized by consolidating the data by shift. For the class of the parameters displayed, the summary rule at this level calls for the range (low and high) to be provided. For example, in FIG. 2, the temperature ranged from a low of 98.6 to a high of 102.6 during the 8:00 to 16:00 shift on January 7 (01/07). The remaining parameters are also of a classification which provides for ranges when collapsed by time.

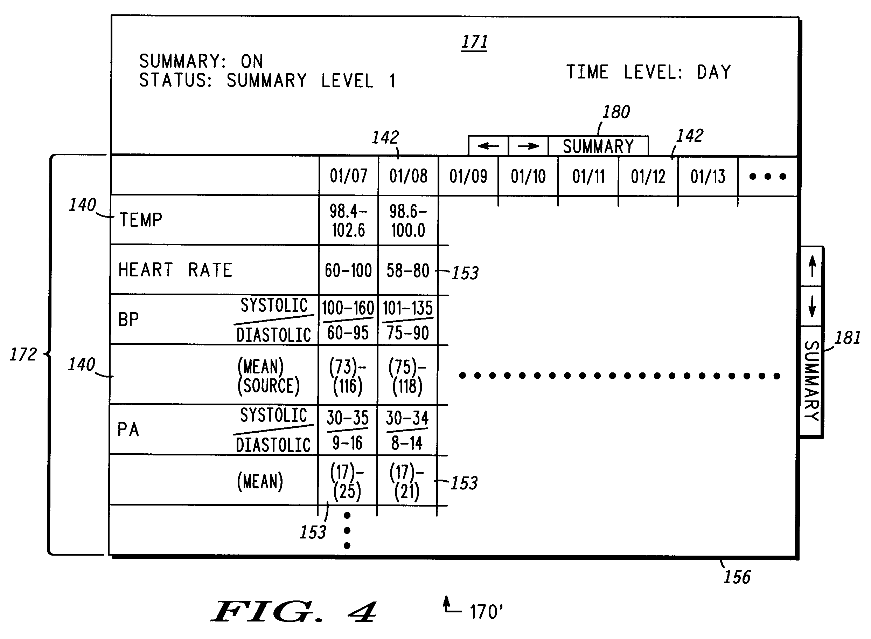

In FIG. 4, the summary of FIG. 3 is further reduced to a daily summary. This can be accomplished by selecting the right arrow of button 180. This summarization may be continued to any time level (e.g. week, month, etc.).

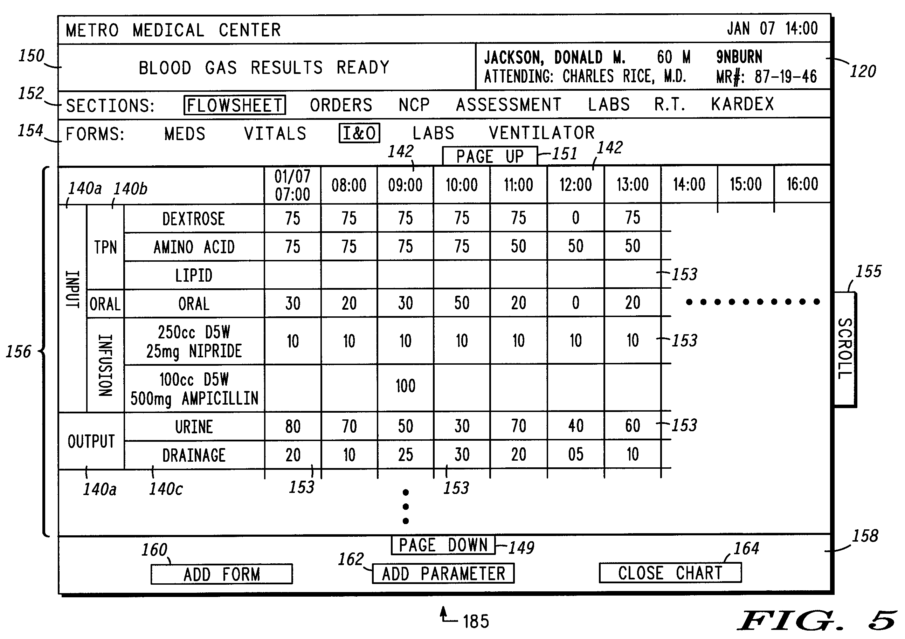

Another example of a flowsheet form is the I&O (input/output) form 185 of FIG. 5. In FIG. 5, row labels 140 are divided into three portions. The first portion, 140a, is a general heading (e.g. input and output). The second portion, 140b, is a group heading (e.g. TPN - triphosphopyridine nucleotide, ORAL, and INFUSION). The third portion, 140c, is the particular item being measured. The output part of form 180 shows urine and drainage.

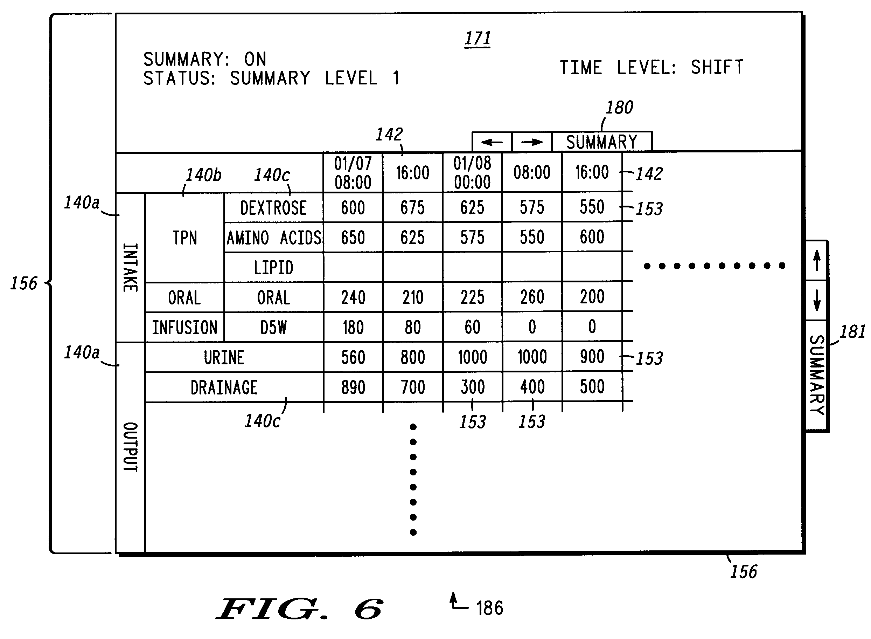

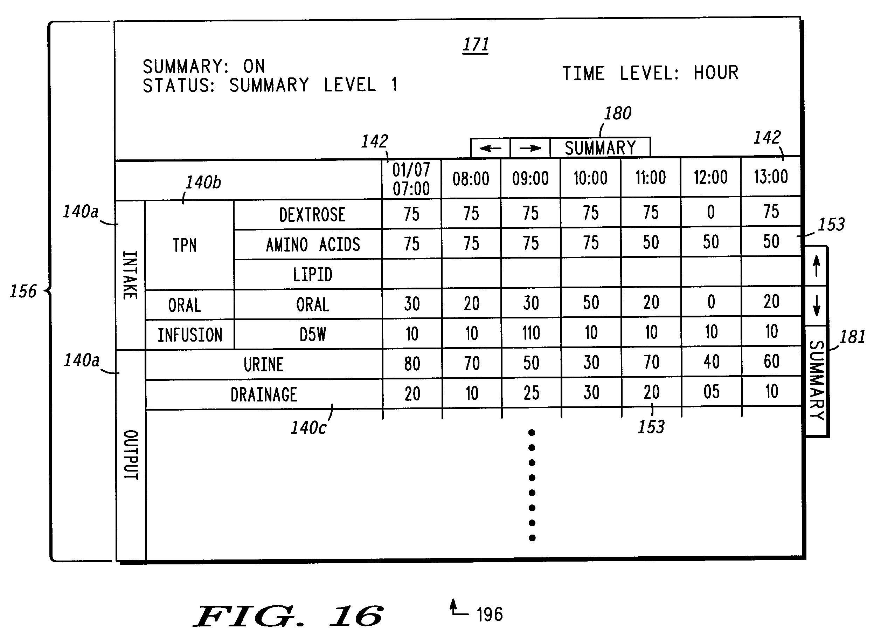

When the summary function is selected, form 186 is provided, FIG. 6. As shown, FIG. 6 is summarized by shift. The primary difference between the summary of FIG. 3 and FIG. 6 is the manner in which the data are summarized. Data of the class of the data of FIG. 3 are summarized by providing ranges over the desired period. Data of the class of the data of FIG. 6 are summarized by providing the sum of the data over the period. Further summaries in the time direction for this class of data will continue to sum the individual entries. In addition, the two instances of D5W have been combined into one entry. This is a spatial or parameter summary and will be discussed further below in reference to FIG. 16.

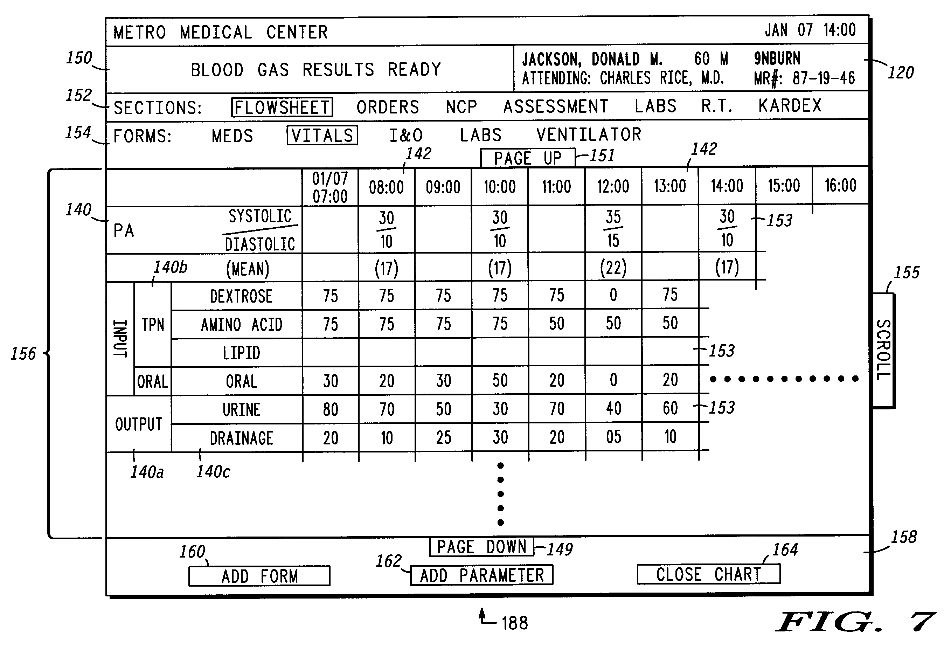

In FIG. 7, a combination display 187 of FIGS. 3 and 5 is illustrated. This type of display will be shown when FIG. 2 is scrolled to the bottom of the vitals form and into the I&O form. In FIG. 2, the PA portion of the vitals form is illustrated with the I&O form of FIG. 5.

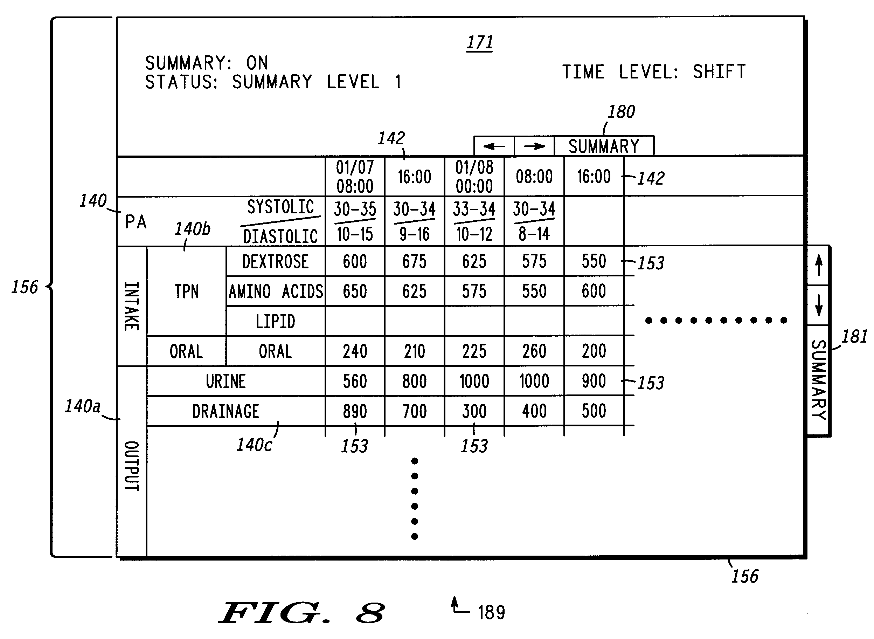

In FIG. 8, the summaries are shown for the data of FIG. 7. In this display, the data are summarized using two different summary rules since the data are two different classes. The vitals data are again summarized by providing ranges and the I&O data are summarized by summing the data. Again, these data are summarized in the time dimension. It should be noted that the summary rule used to produce the summary data is dependent upon the data class and that mixed classes can exist in one form.

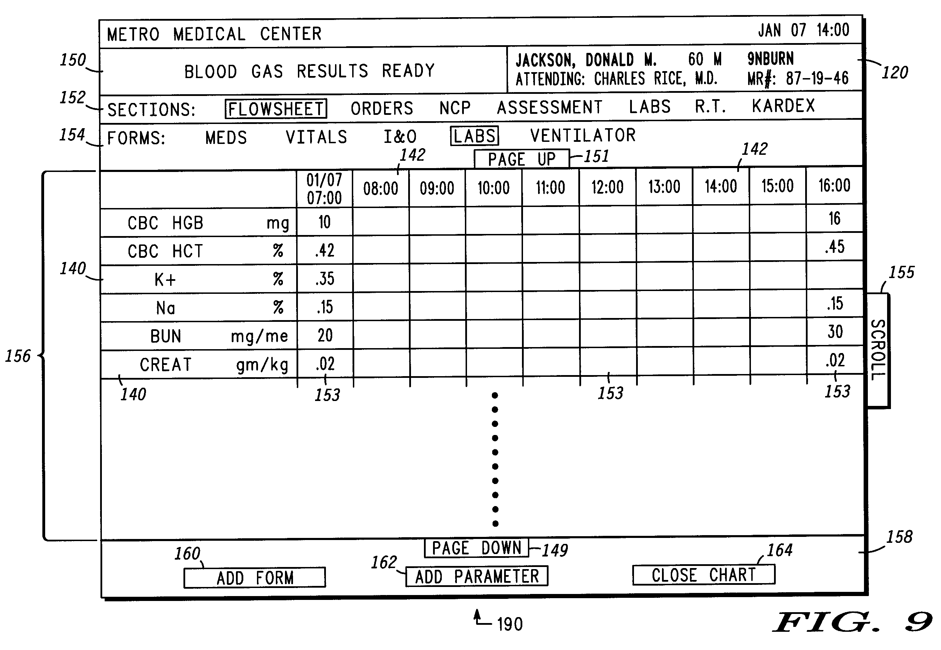

A lab form for the flowsheet section is illustrated as screen display 190 of FIG. 9. The lab form illustrates the results of the lab test for CBC/HGB (Complete Blood Count/HemoGloBin), CBC/HCT (Complete Blood Count/HematoCrit), K+ (Potassium), Na (Sodium), BUN (Blood Urea Nitrogen), and CREAT (CREATinine). As illustrated, these lab tests were reported at 08:00 and 16:00 hours.

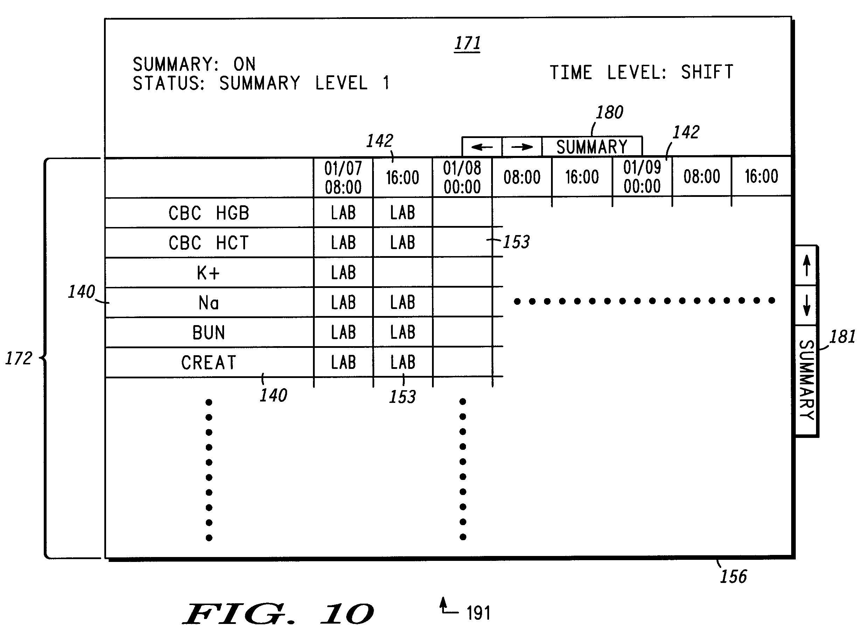

A summary of the lab data is displayed in FIG. 10. For this class of data, the time summary indicates whether or not data is present by placing the word "LAB" (or other appropriate indicator) in cells 153.

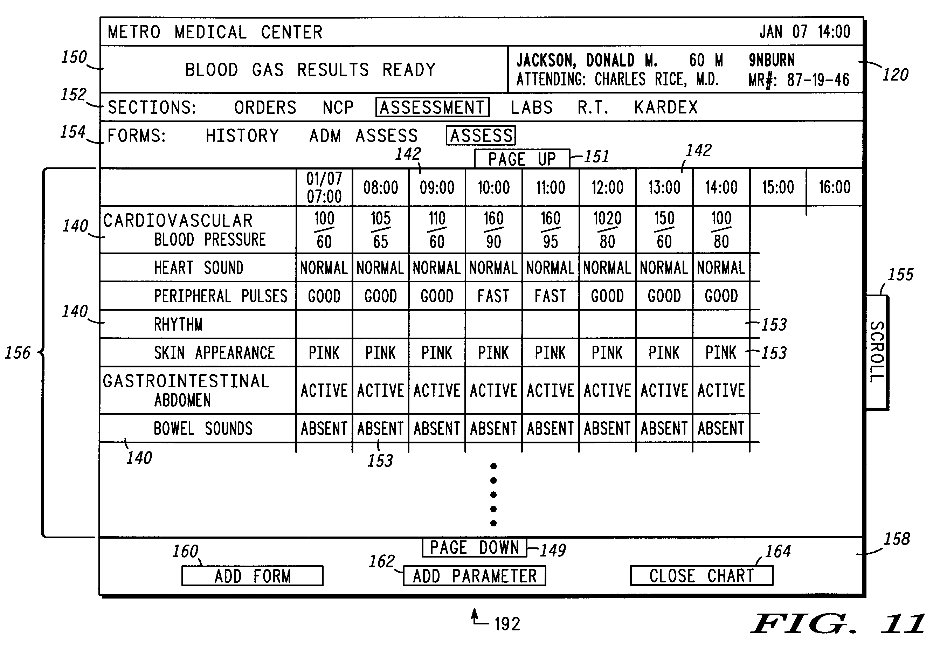

Referring now to FIG. 11, a display screen, generally designated 192, illustrating an ASSESS form of an Assessment section is provided. In this form assessments have been conducted hourly by the health care provider and entered into the computer system. In FIG. 11, cardiovascular assessments are provided for blood pressure, heart sound, peripheral pulses, rhythm, and skin appearance. Gastrointestinal assessments are provided for abdomen and bowel sounds.

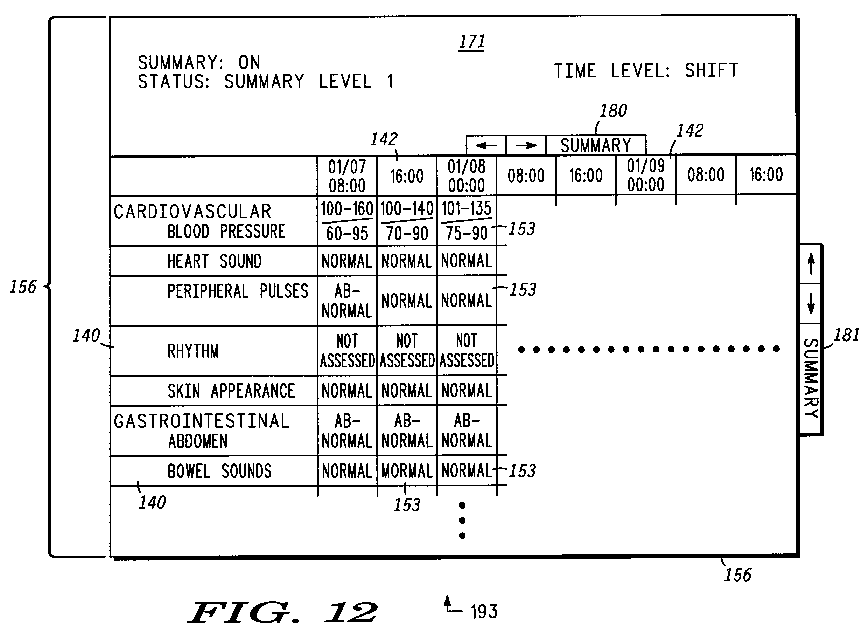

The summary of the ASSESS form is provided in FIG. 12. As shown in FIG. 3, the data for blood pressure is in a class which, when summarized, presents a range. Therefore, a range of blood pressure is provided for the time summary in FIG. 12. The remainder of the data shown in FIG. 11 is of a class in which the summary rule will indicate that either the data for the time period is normal, abnormal, or not assessed. Again, the summary provided in FIG. 12 can be summarized further in time by using button 180.

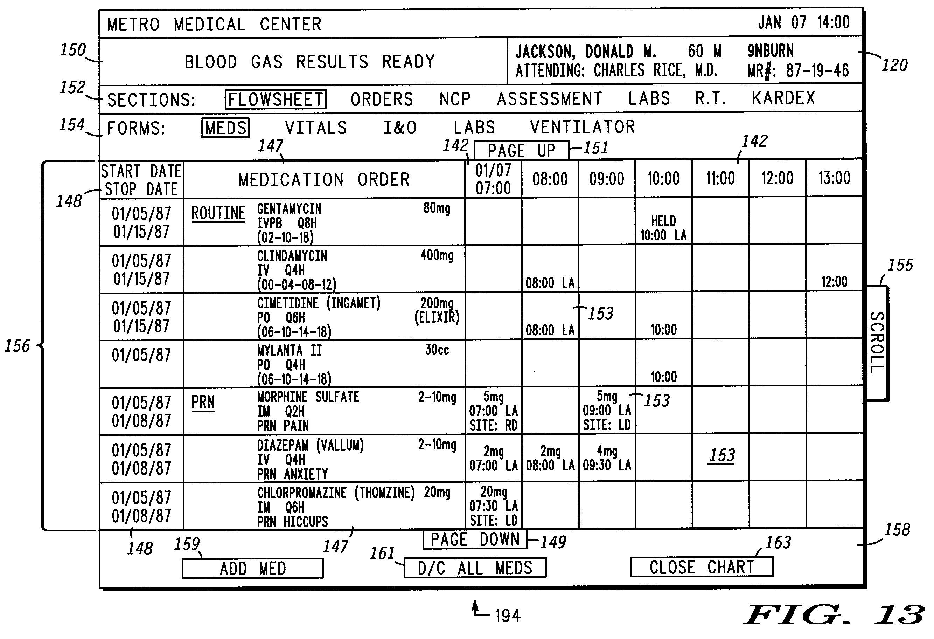

In FIG. 13, a graphical representation of a screen display, generally designated 194, illustrating a MEDS form of the FLOWSHEET section is provided. This form has a start/stop date column 148 and a medication order column 147 in addition to the standard time headers 142. As shown, the medication may specify a set amount (e.g. 80 mg for Gentamycin) or a range (e.g. 2-10 mg for Morphine Sulfate). A full description of this is provided in U.S. Patent No. 4,878,175.

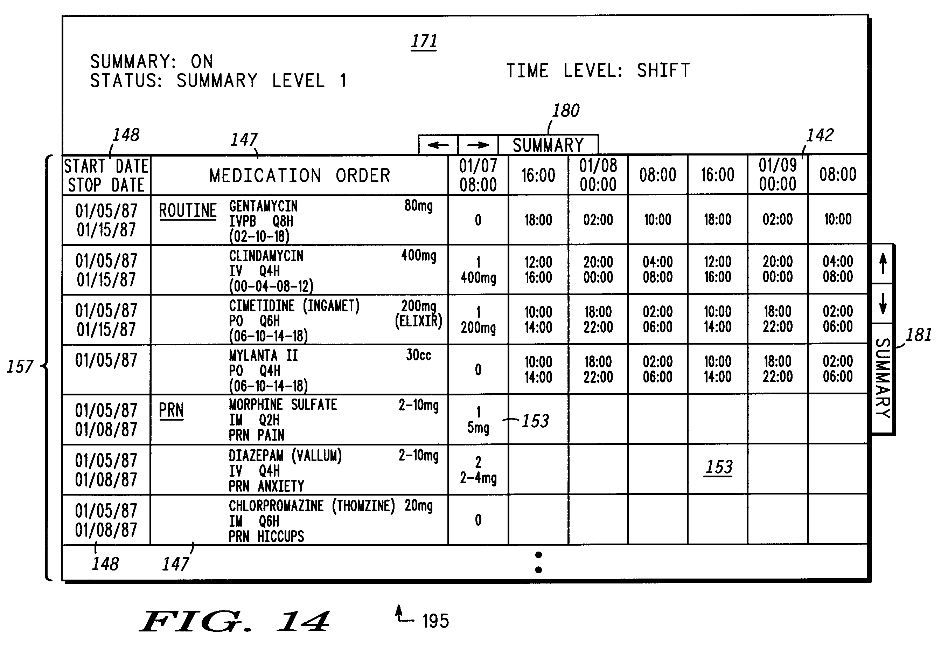

The summary of the data of FIG. 13 is provided in a display 195 of FIG. 14. As illustrated, the summary rule for this class of data summarizes the data by providing the number of dosages and the total of doses. As shown in the 01/07, 8:00, Diazepam cell, two doses were given during this time period and the dosages ranged form 2 to 4 mg. Since medications are scheduled items, another example of a summarization rule would be to only show the times medications are scheduled and not yet administered.

Other examples of time summarization rules include displaying the minimum, maximum, first, count, mode, median, and scheduled items for the given time period.

Until this point, the summarization examples have demonstrated summarization in the time dimension. Summarization may also be performed in the spatial, or parameter, dimension.

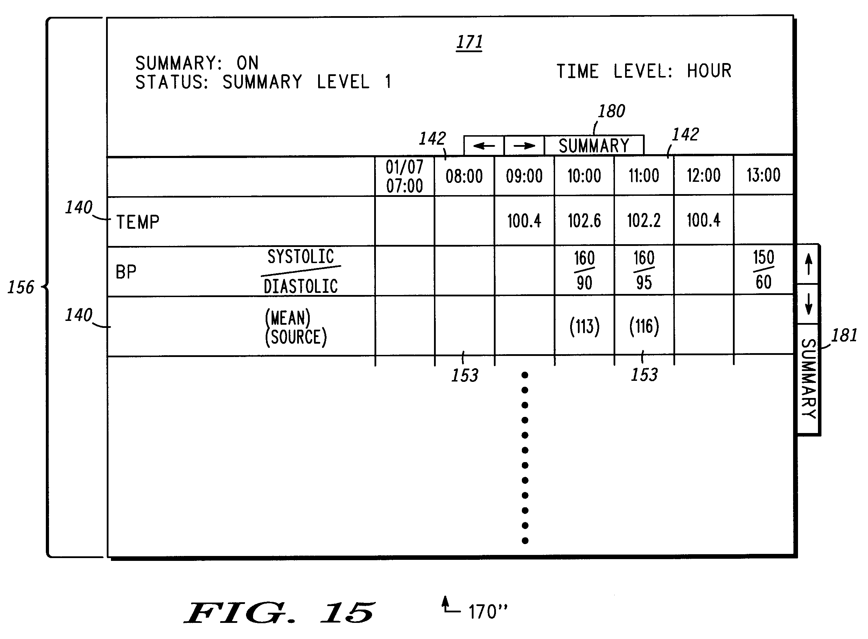

Referring to FIG. 15, the vital signs of FIG. 2 are shown summarized in the parameter direction. Here, the summary rule for this class of data only displays the parameter if the data was abnormal. Therefore, since the PA and Heart Rate parameters were within normal range, they are not displayed in the summary. The values to which the data are compared to make the above normal/abnormal decisions are derived from one or more of the following methods. The most basic method is to preprogram standard normal values into the information system. The actual data are then compared to these values to determine if they are abnormal. A second method would be to input normal values for the patient. These input values may be derived from a patient's past history or based upon a treatment the patient is receiving (e.g. medication). The input values could be used to override the programmed normal values. A third method is to dynamically determine the normal values based upon the actual monitored values. For example, if the patient's heart rate averages 87 bpm (beats per minute) over an extended time period, this may be used to set a normal range of ± 5, or 82-92 bpm.

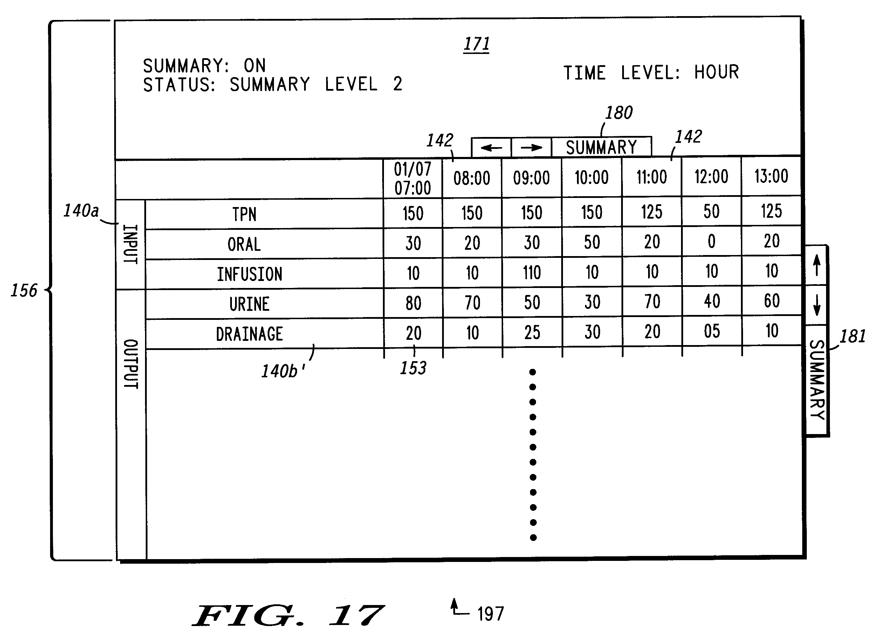

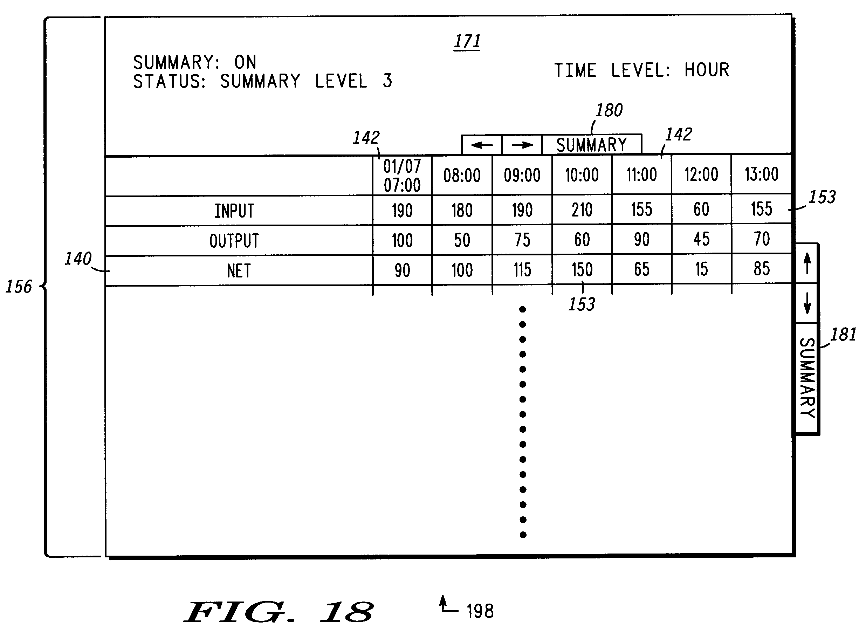

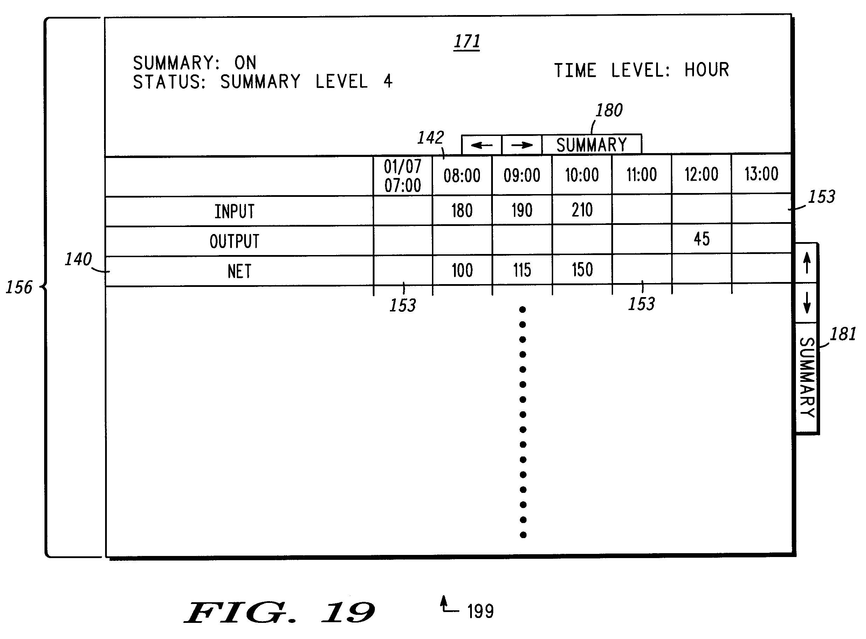

As illustrated in FIG. 6, a summary of the I&O form consists of combining different instances of the same fluid. This is again illustrated in FIG. 16 where summary level 1 is again shown, only this time with the time level set to hourly. Here the classification of the data for the two D5W parameters has resulted in their combination entry. If the summary of FIG. 16 is further summarized by parameter (e.g. by selecting button 181), screen 197 of FIG. 17 is provided. Here the data has been further summarized by the category of the class of data. All of the TPN entries have been combined into one row. If the summary is again selected, the status changes to level 3 and the categories are collapsed into input, output, and net, FIG. 18. Finally, the highest level for this class of data is reached at summary level 4, FIG. 19. This summary level provides the same rows as in FIG. 18, but the data itself is only displayed if it is abnormal.

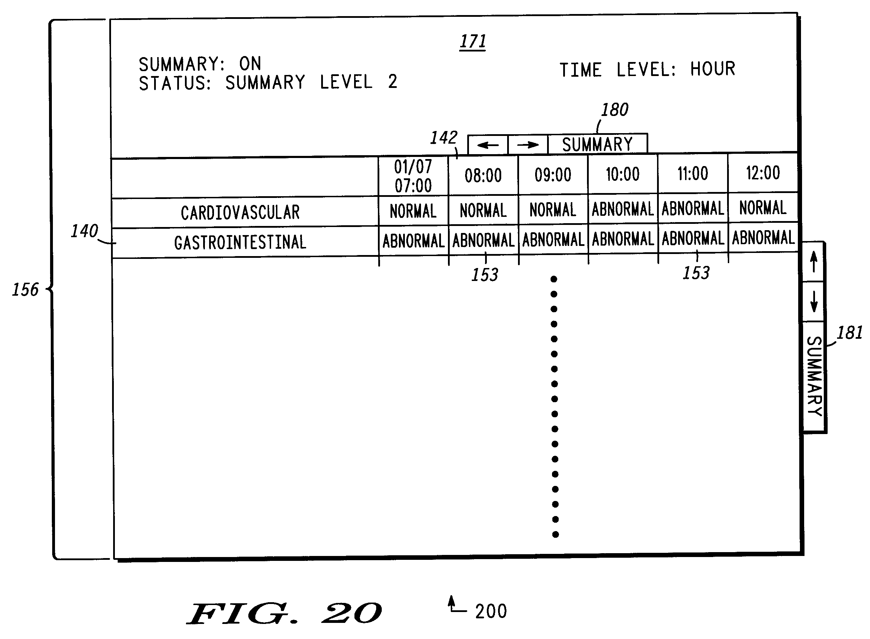

A summary of the ASSESS form of the ASSESSMENT section, FIG. 11, is provided in a display screen 200 of FIG. 20. The status of FIG. 20 is summary level 2. At this level, the individual factors have been collapsed into the class of organ to which they relate. Therefore, the list of parameters of FIG. 11 has been reduced to two (CARDIOVASCULAR and GASTROINTESTINAL) in FIG. 20. As noted earlier, this is summary level 2. Summary level 1 of this class of data would be a repeat of the data shown in FIG. 11. The class of data which comprises the ASSESS form would continue to be collapsed level-by-level until only one general entry were remaining.

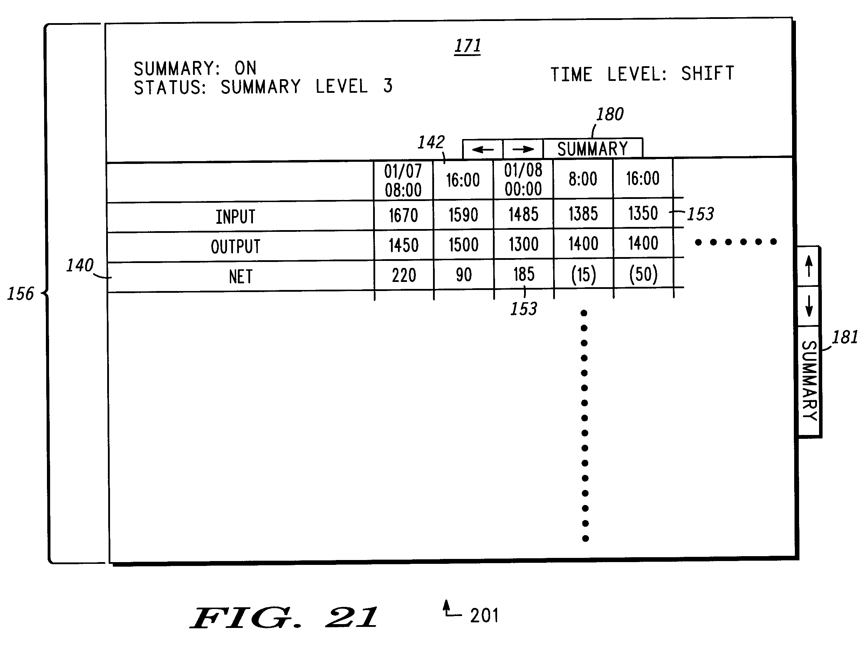

While the preceding has shown summaries in the spatial (parameter) direction, it should be noted that at any spatial level summary, the time summary may be changed and vice versa. An example of this is provided in screen display 201 of FIG. 21. Display 201 is the summary level 3 display of FIG. 18 with the time level from hour to shift.

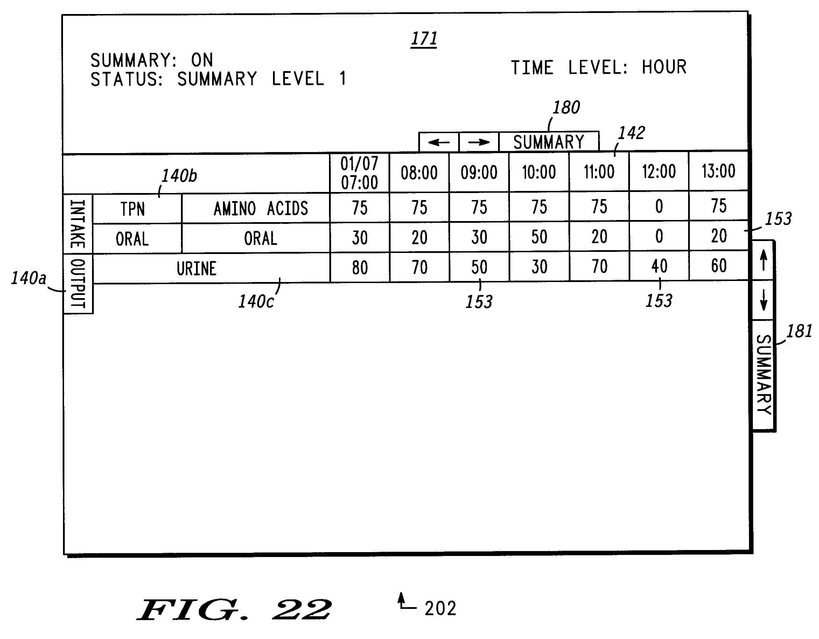

Another aspect of the preferred embodiment permits the user to select the parameters or time intervals which are to be summarized. For example, starting with FIG. 16, the user may wish to view only the amino acids, oral, and urine parameters. By selecting these parameters, the summary will provide screen display 202 of FIG. 22. These parameters may be selected in any of several well known methods. One method of selecting would be to use a cursor to point and "click" on the parameters desired. Another method would be to provide a pop-up menu having a listing of the parameters and selecting the parameters from that listing. It should be noted that the same function may be conducted in the time dimension.

As demonstrated in FIG. 8, more than one class of parameter can be provided on the same display screen. When this occurs, it is not unlikely for one class of parameter to have a different number of summary levels than the other parameter. When this occurs, the parameter with the fewer number of summary levels will continue to display at its maximum summary level. Once the summary rule having the most summary levels reaches its maximum level, the display can be set to prevent further increases in the summary level. Likewise, as the user moves back down through the summary levels, the rules having fewer levels will remain at maximum summary until a level is reached equal to their maximum summary level. After that point, all of the parameters will move down through the summary levels until the summary is eventually exited.

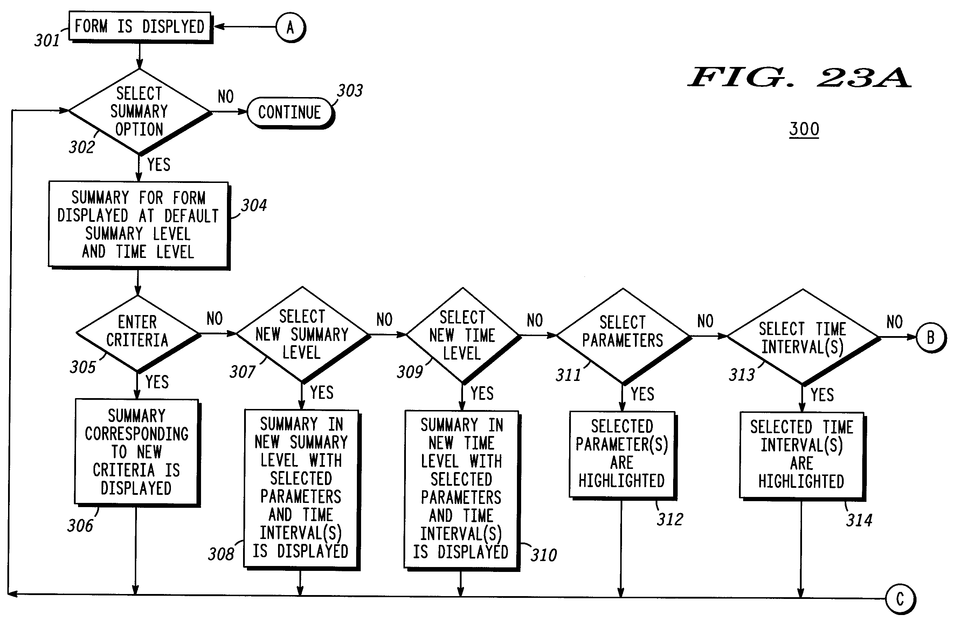

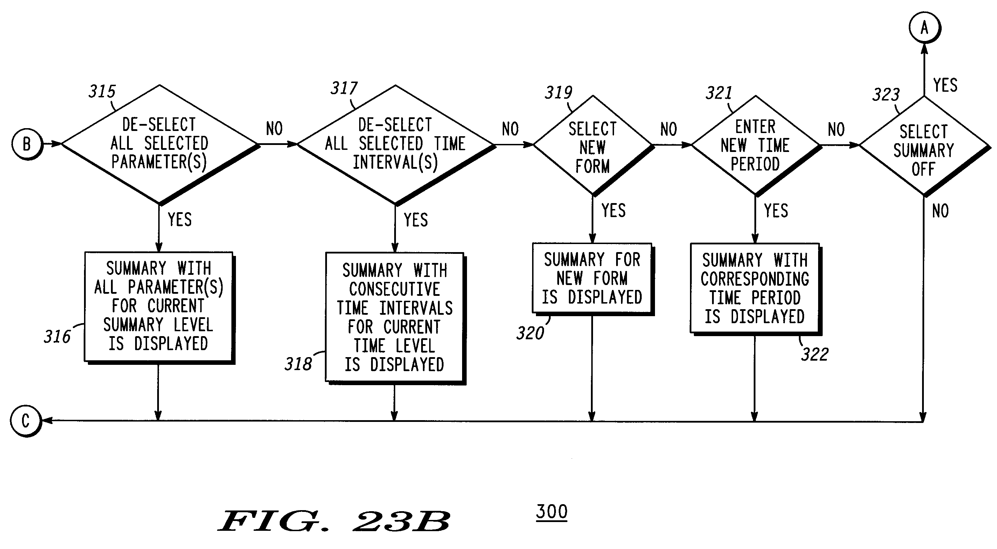

Referring now to FIG. 23, a block diagram of a flow chart of a process, generally designated 300, illustrating the user interface process is provided. Process 300 commences at step 301 where a form is displayed. Process 300 then continues to decision step 302 where it is determined if a summary option has been selected. If the summary option has not been selected, then the process continues. If the summary option is selected, process 300 moves to step 304 and displays the summary form at default summary and time levels.

Following step 304, process 300 moves through a number of decision steps used by the user to tailor the summary. The next step is decision step 305 where it is determined if the user has changed any of the summary criteria. These criteria may be a change in the normal data to which the actual data are compared to determine normal/abnormal results. If different criteria have been entered, then a new summary display is provided using the new criteria, step 306. Following this, process 300 loops back to decision step 305.

If there was no new criteria entered, process 300 then determines, in decision step 307, if a new summary level has been selected. If a new summary level has been selected, then the process generates a new summary form using the selected summary level of the summary rule, step 308. The process then returns to decision step 305.

If there was no new summary level selected, then process 300 determines if a new time level was selected, decision step 309. If a new time level was selected, a new summary form is generated using the new time level, step 310. This process continues through decision steps 311 (selecting specific parameters) and 313 (selecting time intervals).

When decision step 315 is reached, process 300 determines if selected parameters have been de-selected. If they have, then all of the parameters are again displayed in display step 316. Similarly, any time intervals selected may be de-selected in steps 317 and 318.

In decision step 319, it is determined if a new form has been selected. This can be accomplished by selecting a new section and/or a new form from areas 152 and 154, respectively. If a new form has been selected, it is displayed at the current summary level. Process 300 then loops back to decision step 305.

Process 300 then reaches decision step 321 where it is determined if a new time period has been entered. If it has, a new summary form for the specified time period is displayed, step 322. It should be noted here that selecting a new time period is different from step 313 where specific time intervals are selected.

If no new time period is entered, process 300 moves to step 323 where it is determined if the summary function is turned off. If it is, then the process loops back to step 301 where the form is displayed. If the summary was not turned off, process 300 loops back to step 305.

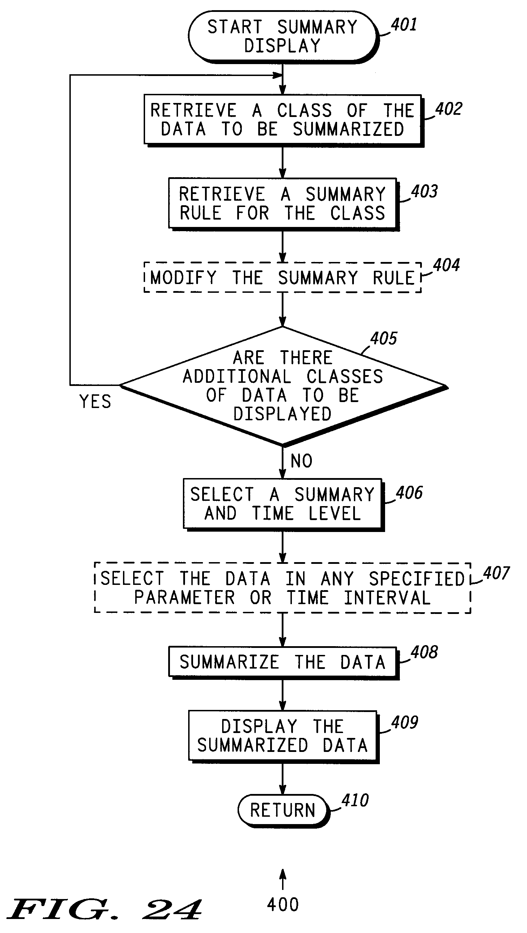

The method of generating the summary referred to in process 300 is provided in the flow chart of FIG. 24, generally designated 400. Process 400 starts at step 401 when a command is received to generate a summary display. Process 400 then moves to step 401 where a class of the data is retrieved, and to step 403 where a summary rule for that class is retrieved. Both of these items are retrieved from the storage means of the computer system.

Following step 403 is an optional step 404 illustrated by a dashed box. In step 404, if any special directions have been entered by the user (e.g. in criteria decision step 305 of FIG. 23), the summary rules are modified to comply.

Process 400 then determines if there are any additional classes of data to be displayed, decision step 405. If there are additional data classes, then process 400 loops back and repeats steps 402-404 until all of the summary rules have been retrieved.

Once all of the summary rules are retrieved, a summary level and time level are determined, step 406. Specific parameters or time intervals may then be selected for summary, step 407. Again, this step is optional and corresponds to steps 312 and 314 of FIG. 23.

The data are then summarized, step 408, using the summary rule for that class at that summary level, and the summarized data is displayed, step 409. Process 400 then returns, step 410.

Therefore, a method has been shown which provides a method of summarizing patient data to provide the health care provider with data in a form which will reduce the time needed to review the patient's records. In addition, these summaries are organized to progressively move information through several levels of summarization.

Thus, it will be apparent to one skilled in the art that there has been provided a data cell that fully satisfies the objects, aims, and advantages set forth above.

While the invention has been described in conjunction with specific embodiments thereof, it is evident that many alterations, modifications, and variations will be apparent to those skilled in the art in light of the foregoing description. Accordingly, it is intended to embrace all such alterations, modifications, and variations in the appended claims.

## Claims

1. In an information based computer system (FIG. 1) having a means for storing a plurality of data (12), each having a class associated therewith, and each class having a summary rule associated therewith, a method for providing a summary display of said data comprising the steps of:
A) retrieving a portion of a first class of data of said plurality of data from said means for storing (402, FIG. 24);
B) retrieving a first summary rule for said first class of data from said means for storing (403), said first summary rule having a plurality of summary levels associated therewith;
C) repeating steps A and B for second through Nth portions of said data in said plurality of data (405), wherein N is a positive integer;
D) selecting one of said plurality of summary levels for each of said summary rules associated with said N data portions (406);
E) summarizing said N data portions (408) based upon the corresponding selected summary level of its associated summary rule, forming summarized data for each of said data portions; and
F) displaying said summarized data (409).

2. The method of claim 1 further comprising the step of modifying said summary rule (404) associated with each of said data portions before step (E).

3. The method of claim 1 wherein said summary rule associated with each of said data portions performs at least one of the following operations:
G) sums the data within a designated time interval;
H) determines a range of said data portion within a designated time interval;
I) provides a normal/abnormal indicator (FIG. 12) for said data portion over a designated time interval;
J) provides a present/not present indicator for said data portion over a designated time interval;
K) determines a minimum value of said data portion within a designated time interval;
L) determines a maximum of said data portion within a designated time interval;
M) determines a total of said data portion within a designated time interval;
N) determines a first data entry of said data portion within a designated time interval;
O) determines a count of said data within said data portion within a designated time interval;
P) determines a mode of said data portion within a designated time interval;
Q) determines a median of said data portion within a designated time interval; or
R) determines which of said data portions are scheduled for a predetermined activity within a designated time interval.
